# EUROPEAN PATENT SPECIFICATION

(11) **EP 0 572 735 B1**
(45) Date of publication and mention of the grant of the patent: **25.11.1998**
(21) Application number: 92305124.7
(22) Date of filing: 04.06.1992
(51) Int. Cl.: C12Q 1/68

(54) **Nucleic acid probes for the detection of Mycoplasma fermentans**
Nukleinsäuresonden zum Nachweis von Mycoplasma fermentans
Sondes d'acide nucléique pour la détection de Mycoplasma fermentans

(43) Date of publication of application: 08.12.1993
(73) Proprietor: VYSIS, Inc., Downers Grove, Illinois 60515-5424 (US)
(72) Inventor: Weisburg, William Greene, Milford, MA 01757 (US); Pelletier, Dale Adam, Brighton, MA 02135 (US)
(74) Representative: Hall, Marina

(56) References cited:
- EP-A- 457 685
- WO-A-88/03957
- THE JOURNAL OF INFECTIOUS DISEASES vol. 165, no. 3, March 1992, CHICAGO US pages 581 - 585 R.E.HAWKINS ET AL.
- RESEARCH IN VIROLOGY vol. 141, 1990, PARIS pages 385 - 395 C.SAILLARD ET AL.
- FEMS MICROBIOLOGY LETTERS vol. 81, 1 June 1991, AMSTERDAM NL pages 37 - 42 A. BLANCHARD ET AL.
- JOURNAL OF BACTERIOLOGY vol. 171, no. 12, December 1989, BALTIMORE US pages 6455 - 6467 W.G.WEISBURG ET AL.

## Description

### Field of the Invention

This invention relates to the detection of Mycoplasma fermentans which has been described as having an association with the pathogenesis of Acquired Immunodeficiency Syndrome (AIDS). The invention features nucleic acids and methods for their use for the specific detection and identification of Mycoplasma fermentans.

The present invention relates to the detection of
Mycoplasma fermentans, which is genetically identical to a recently described virus-like infectious agent (VLIA) temporarily designated "Mycoplasma incognitus", which is found with high frequency in patients with AIDS. More specifically, one embodiment of the present invention features nucleic acid compositions and methods for their use for the specific detection or identification of Mycoplasma fermentans and any genetically identical mycoplasma species in clinical and other samples. The nucleic acid compositions feature nucleotide sequences, from 10 to 250 nucleotides, which are capable of hybridizing preferentially to the rRNA and rDNA of Mycoplasma fermentans.

### Background of the Invention

Mycoplasmas are small wall-less bacteria, primarily isolated from animal sources including humans. There are over 70 members of the genus Mycoplasma, and several related genera which are also characterized as small wall-less bacteria; these are Spiroplasma, Acholeplasma, Ureaplasma, Anaeroplasma, and Asteroleplasma. Only a handful of the species within these genera have been found associated with humans--some presumed to be "normal flora", others occasionally pathogenic, and still others always believed to be clinically significant. Among the mycoplasmas known to be pathogenic, Mycoplasma pneumoniae is historically the most well studied, and it is the major infectious agent of primary atypical pneumonia. Nucleic acid compositions and methods relating to Mycoplasma pneumoniae is the subject of an application filed concurrently herewith USSN 673,686, entitled "Nucleic Acid Probes For The Detection Of Mycoplasma Pneumoniae." At least one inventor is common to that application and the present application.

Three other mycoplasma species, which can be isolated from the human genito-urinary tract, Mycoplasma hominis, Mycoplasma genitalium, and Ureaplasma urealyticum, are somewhat more enigmatic in the clinical implications of the detection of these organisms in the human body. Ureaplasma urealyticum, for example, although it is implicated in significant and serious human morbidity and mortality, may be found in asymptomatic "normal" individuals as well. The three species, Mycoplasma hominis, Mycoplasma genitalium, and Ureaplasma urealyticum may be referred to as the genital mycoplasmas. Nucleic acid compositions and methods relating to genital mycoplasmas are the subject of an application filed concurrently herewith USSN 673,661, entitled "Nucleic Acid Probes For The Detection Of Genital Mycoplasmas." At least one inventor is common to that application and the present application.

In 1989, an additional important pathogenic mycoplasma was reported by S.C. Lo and colleagues at the American Armed Forces Institute of Pathology (Am. Jnl. Trop. Med. Hyg. vol. 40:213:226, 1989, vol 41:364-376, 1989, vol. 41:586-600, 1989). The mycoplasma reported by Lo, temporarily designated "Mycoplasma incognitus" was discovered as a result of a dubious transformation of a cell culture with what was thought to be a viral DNA preparation. Quotation marks are ordinarily used to designate binary bacterial names that are not approved by the governing taxonomic organizations; Mycoplasma incognitus would not be considered an approved name. This mycoplasma, after growth in rich SP-4 media, was the source of various DNA probes and immuno-histochemical test reagents. Testing of samples from AIDS patients, and controls indicated that the mycoplasma VLIA was found in blood and numerous tissues from AIDS patients with high frequency (Lo, et.al. Am Jnl. Trop. Med. Hyg. vol 40, and vol. 41, 1989).

Tests to determine the genetic and serological relatedness of the so-called Virus-Like Infectious Agent, "Mycoplasma incognitus", all point to the designation of Mycoplasma fermentans as the same identical species as the VLIA (Lo, et al. Am. Jnl. Trop. Med. Hyg., vol. 41:586-600, 1989, and Saillard, et al. Res. Virol. vol. 141:385-395, 1990). Polyclonal rabbit antiserum raised against VLIA/"M. incognitus" reacted with only Mycoplasma fermentans and VLIA. In the reciprocal experiments, mule antiserum raised specifically against M. fermentans, reacted only with these same two mycoplasmas (except at very high protein concentrations). Restriction enzyme patterns, Southern blot analysis, and similar restriction fragment length polymorphism revealed a high degree of identity between "Mycoplasma incognitus" and Mycoplasma fermentans (Lo, et al., Am. Jnl. Trop. Med. Hyg. vol. 4, pg. 586-600, 1989). Saillard et al. report that DNA-DNA homology between VLIA and M. fermentans is approximately 90%. This is fully consistent with Mycoplasma fermentans and "Mycoplasma incognitus" being the same species, and therefore identical at the level of sequence of the ribosomal RNA. By all criteria, genetic and serological, "Mycoplasma incognitus" is a strain of Mycoplasma fermentans, and should be designated as Mycoplasma fermentans VLIA strain. As used herein, Mycoplasma fermentans, Mycoplasma incognitus, VLIA, and Lo's Virus-Like Infectious Agent are synonyms for a single naturally occurring biological entity, correctly known as Mycoplasma fermentans.

In separate reports found in the scientific literature, international AIDS symposia, and the newspapers, L. Montagnier and colleagues at the Institut Pasteur report discovery of mycoplasma bacteria associated with the pathogenesis of AIDS. In one report on the protective activity of antibacterial agents against the cytopathic effects of Human Immunodeficiency Virus (HIV), they speculate that a cofactor, "Mycoplasmas are the likely candidates," is responsible for HIV-induced cell lysis (Lemaitre, et al. Res. Virol. vol. 141:5-16). At the Sixth International Conference on AIDS in 1990, Montagnier reported this mycoplasma to be Mycoplasma pirum (New york Times June 22, 1990 and June 26, 1990).

Additional discussions and assessments of the credibility of the mycoplasma cofactor theory of AIDS have been published in the American Society for Microbiology News (Baseman and Quackenbush, ASM News, 1990), the editor's page of the American Journal of Tropical Medicine and Hygiene (Am. J. Trop. Med. Hyg., vol 42:399-402, 1990), and Science Magazine (K. Wright, vol. 248, 1990). Some additional reports have suggested that the AIDS-associated mycoplasma may be Mycoplasma genitalium. Nucleic acid probes, compositions, and methods for the specific detection of Mycoplasma genitalium may be found in copending USSN 673,661 entitled "Nucleic Acid Probes for the detection of Genital Mycoplasmas," filed concurrently herewith.

The mycoplasmas, such as the ones described above, are fastidious organisms, requiring complex culture media containing peptone, yeast extract, expensive animal sera, and sterol. Growth is relatively slow and reaches low cell densities compared to most bacteria. In addition, atmospheric conditions for cell growth require the addition of carbon dioxide. For these reasons, most clinical laboratories are unable to perform culture isolation of mycoplasmas, and consequently are left with no real ability to diagnose the presence of these important pathogenic bacteria. In fact, there has yet to be a confirmed isolation of M. fermentans VLIA except for the confusing original transformation experiments. There is only one anecdotal report of isolation of Mycoplasma pirum from human, and only a few reports of Mycoplasma genitalium isolation.

Given that mycoplasmas lack cell walls, antibiotics that target the bacterial cell wall, such as penicillin, have no anti-mycoplasma activity. Consequently, it is of importance for a physician to make a diagnosis of the presence of this bacterium, particularly if the clinical presentation is predictive, and prescribe the appropriate antibiotic.

Kohne et al. (Biophysical Journal 8:1104-1118, 1968) discuss a method for preparing probes to rRNA sequences. Kohne et al. do not provide the teaching necessary to make probes to detect Mycoplasma fermentans or Mycoplasma pirum.

Pace and Campbell (Journal of Bacteriology 107:543-547, 1971) discuss the homology of ribosomal ribonucleic acids from diverse bacterial species and a hybridization method for quantitating such homology levels.

Similarly, Sogin, Sogin and Woese (Journal of Molecular Evolution 1:173-184, 1972) discuss the theoretical and practical aspects of using primary structural characterization of different ribosomal RNA molecules for evaluating phylogenetic relationships. Fox, Pechman and Woese (International Journal of Systematic Bacteriology 27:44-57, 1977) discuss the comparative cataloging of 16S ribosomal RNAs as an approach to prokaryotic systematics. Sogin et al., Fox et al. and Pace and Campbell do not provide specific probes useful in assays for detecting the presence and abundance of Mycoplasma fermentans, Mycoplasma pirum or VLIA in samples.

Hogan, et al. (International Patent Application, Publication Number WO 88/03957) describe five probes for the specific detection of Mycoplasma pneumoniae, but fail with respect to the specific detection of Mycoplasma fermentans, Mycoplasma pirum or VLIA.

Woese, Maniloff, Zablen (Proc. Natl. Acad. Sci. USA vol. 77, 1980) examined the partial sequences of selected mycoplasma 16S rRNA, and define the concept of mycoplasma sequence "signature", but fail to teach the art of probe design. They do not discuss M. fermentans.

Rogers, et al. (Proc. Natl. Acad. Sci. USA, vol. 82, 1985) discuss sequences of 5S rRNA, but fail to teach the art of probe design. They do not mention M. fermentans.

Weisburg, et al. (Jnl. Bacteriology, vol. 171, 1989) discuss 16S rRNA sequences of numerous mycoplasmas including Mycoplasma fermentans and Mycoplasma pirum, but do not discuss the VLIA or any aspect of probe design for any mycoplasma.

EP-A-0 457 685 describes the detection of mycoplasmas which are present in the blood of patients infected by HIV. The disclosure also relates to detectors which are specific to the mycoplasma group, but which are not specific to a given species. Once a blood sample has been processed according to methods described in EP-A-0 457 685, the DNA of a mycoplasma in the sample is detected using a detector containing a DNA sequence which is derived from a sequence strongly conserved in mycoplasma rRNA's.

Ribosomes are of profound importance to all organisms because they serve as the only known means of translating genetic information into cellular proteins, the main structural and catalytic elements of life. A clear manifestation of this importance is the observation that all cells have ribosomes.

Bacterial ribosomes contain three distinct RNA molecules which, at least in Escherichia coli, are referred to as 5S, 16S and 23S rRNAs. In eukaryotic organisms, there are four distinct rRNA species, generally referred to as 5S, 18S, 28S, and 5.8S. These names historically are related to the size of the RNA molecules, as determined by their sedimentation rate. In actuality, however, ribosomal RNA molecules vary substantially in size between organisms. Nonetheless, 5S, 16S, and 23S rRNA are commonly used as generic names for the homologous RNA molecules in any bacterium, including the mycoplasmas, and this convention will be continued herein.

As used herein, probe(s) refer to synthetic or biologically produced nucleic acids (DNA or RNA) which, by design or selection, contain specific nucleotide sequences that allow them to hybridize, under hybridization conditions, preferentially to target nucleic acid sequences. The term "preferentially" is used in a relative sense; one hybridization reaction product can be more stable than another under identical conditions.

In addition to their hybridization properties, probes also may contain certain constituents that pertain to their proper or optimal functioning under particular assay conditions. For example, probes may be modified to improve their resistance to nuclease degradation (e.g. by end capping), to carry detection ligands (e.g. fluorescein, biotin, and avidin), to facilitate direct or indirect detection (³²P, and fluorescent and chemiluminescent agents) or to facilitate their capture onto a solid support (e.g., homopolymer "tails"). Such modifications are elaborations on the basic probe function which is its ability to usefully discriminate between target and non-target organisms in a hybridization assay.

Hybridization is the process by which two partially or completely complementary strands of nucleic acid are allowed to come together in an antiparallel fashion (one oriented 5' to 3', the other 3' to 5') to form a double-stranded nucleic acid with specific and stable hydrogen bonds, following explicit rules pertaining to which nucleic acid bases may pair with one another. The high specificity of probes relies on the low statistical probability of unique sequences occurring at random as dictated by the multiplicative product of their individual probabilities. A minimum of ten nucleotides are necessary in order to statistically obtain specificity and form stable hybridization products. A maximum of 250 nucleotides represents an upper limit of nucleotides in which reaction parameters can be adjusted to determine mismatched sequences and preferential hybridization.

Hybridization conditions are defined by the base composition of the probe/target duplex, as well as by the level and geometry of mispairing between the two nucleic acids. Normal hybridization conditions for nucleic acid of approximately 10 to 250 nucleotides would include a temperature of approximately 60°C in the presence of 1.08 M sodium chloride, 60 mM sodium phosphate and 6 mM ethylenediamine tetraacetic acid (pH 7.4).

Hybridization conditions are easily modified to suit nucleic acid of differing sequences. Factors which may influence the hybridization conditions for a particular nucleic acid composition are base composition of the probe/target duplex, as well as by the level and geometry of mispairing between the two nucleic acids.

Reaction parameters which are commonly adjusted are the concentration and type of ionic species present in the hybridization solution, the types and concentrations of denaturing agents present, and the temperature of hybridization. Generally, as hybridization conditions become more stringent, or less favorable for hybridization, longer probes are required to form stable hybrids.

### Summary of the Invention

The present invention features nucleic acids and nucleic acid sets and methods for the specific detection or identification of Mycoplasma fermentans. As used herein, the term "Mycoplasma fermentans" an "virus like infectious agent" (VLIA) are used synonymously.

Embodiments of the present invention feature nucleic acids capable of hybridizing to regions of the 16S rRNA of Mycoplasma fermentans. The regions of particular interest consist of regions bounded by nucleotide location numbers 50 to 100, 130 to 165, 160 to 200, and 420 to 470. The nucleic acids are useful in detecting Mycoplasma fermentans as they selectively hybridize to the 16S rRNA of Mycoplasma fermentans instead of hybridizing to rRNA or rDNA of other mycoplasmas or non-mycoplasma bacteria.

Preferably, the nucleic acid is complementary to or homologous with a sequence comprising any ten consecutive nucleotides within the sequence of probe 2190, 2193, 2261 or 2273.

A further embodiment of the present invention features an article of manufacture for the detection of Mycoplasma fermentans, The article of manufacture comprises a set of nucleic acids comprising at least two nucleic acids. Each nucleic acid has 10 to 250 nucleotides and different base sequence composition. Each nucleic acid is complementary to or homologous with at least 90% of a sequence comprising any ten consecutive nucleotides selected from the group of sequences defined by probes 2190, 2193, 2261 and 2273, A set of nucleic acids is particularly suited for detecting Mycoplasma fermentans in a two-probe, sandwich format assay.

A further embodiment of the present invention features a methods for detecting the presence of Mycoplasma fermentans. The method comprises the step of contacting the sample with at least one nucleic acid. The nucleic acid has approximately 10 to 250 nucleotides capable and is of hybridizing preferentially to Mycoplasma fermentans rRNA or rDNA. The method includes the step of imposing hybridization conditions on the sample to allow the nucleic acid to bind preferentially to Mycoplasma fermentans rRNA or rDNA to form nucleic acid complexes. Finally, the method comprises detecting the nucleic acid complexes as an indication of the presence of Mycoplasma fermentans.

Preferably, the nucleic acid of the present method is complementary to or homologous with at least 90% of a sequence comprising any ten consecutive nucleotides selected from the group of sequences defined by probes 2190, 2193, 2261 and 2273.

A further embodiment of the present invention includes a kit for detecting Mycoplasma fermentans. The kit comprises a nucleic acid having 10 ta 250 nucleotides and capable of hybridizing to rRNA or rDNA of Mycoplasma fermentans in preference to rRNA and rDNA of non-Mycoplasma fermentans microorganisms and humans. Typically, kits are comprised of reagents, compositions, instructions, disposable hardware and suitable packaging to allow marketing in a convenient assembly.

The nucleic acids, kits, and methods of the present invention provide the basis for development of valuable nucleic acid hybridization assays for the specific detection of Mycoplasma fermentans, The type of samples which may be encountered include sputum, throat swabs, blood, urine, cerebrospinal fluid, skin, biopsy, saliva, synovial fluid, bronchial wash, bronchial lavage, or other tissue or fluid samples from human patients or veterinary subjects. The discovery that probes could be generated with the extraordinary inclusivity and exclusivity characteristics of the present invention with respect to the detection of Mycoplasma fermentans, without necessarily incurring cross-reactivity between other mycoplasma species, and other bacteria, was unexpected.

### Brief Description of the Tables and Figures

Further understanding of the principles and aspects of the present invention may be made by reference to the Tables and Figures.

Table 1 describes the physical structure of the probes.

Table 2 describes the hybridization behavior of the probes towards the panel of clinically and environmentally representative mycoplasma species.

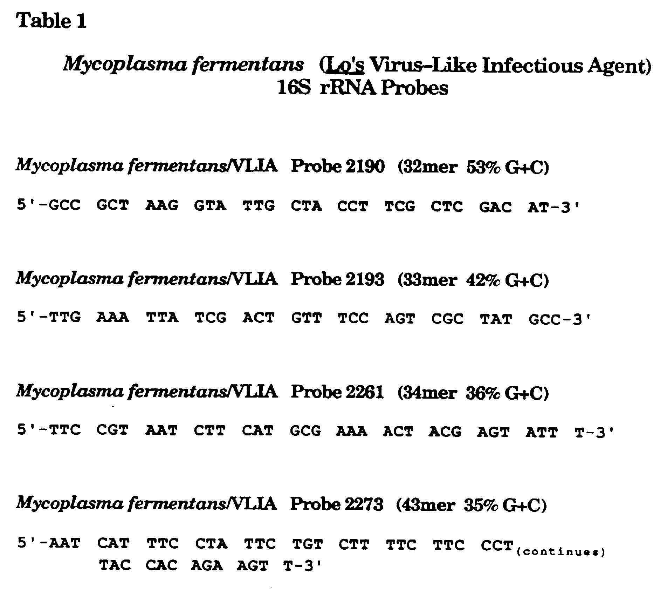

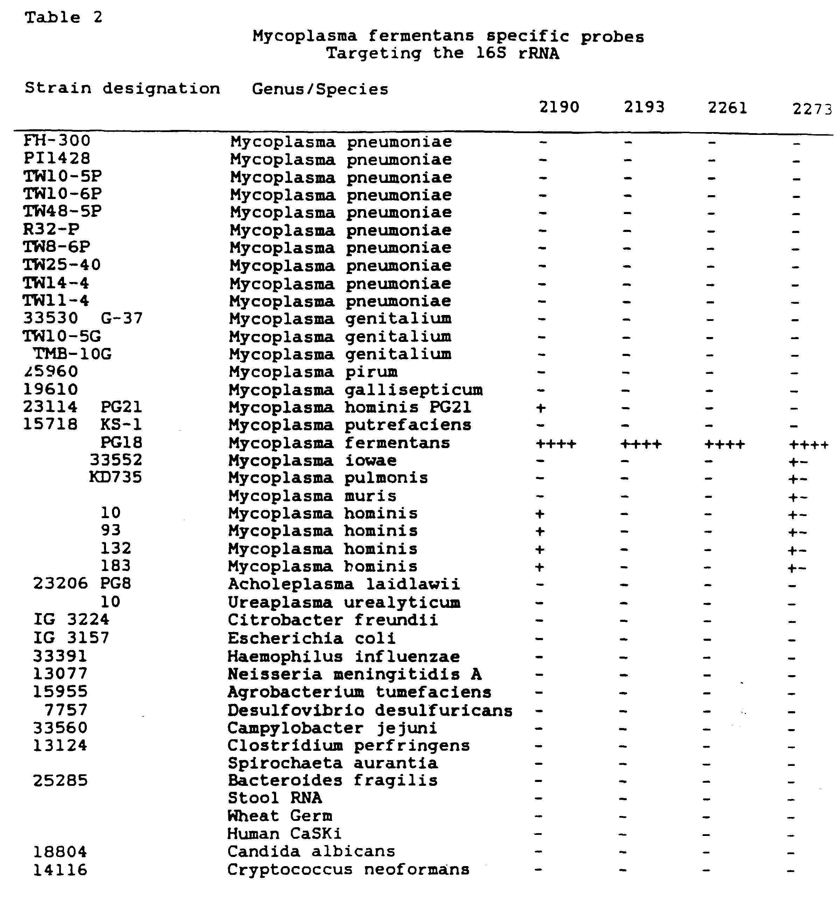

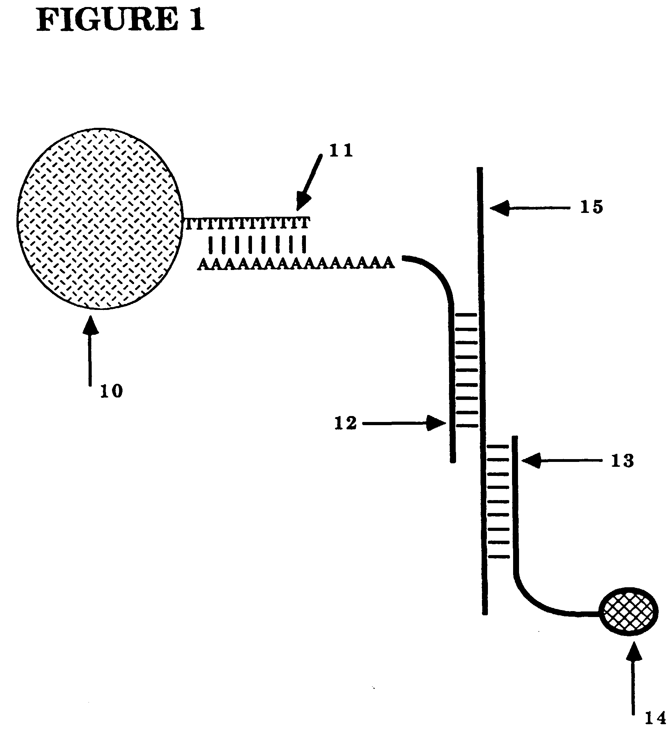

Figure 1 is a schematic representation of a sandwich assay utilizing a capture probe, detector probe and support.

### Detailed Description of the Invention

The present invention will be described in detail with respect to nucleic acids having utility as probes for the identification of Mycoplasma fermentans. The present discussion will highlight probe development strategy and examples featuring dot blot analysis, sandwich assay formats, in situ applications and amplification techniques for utilizing the nucleic acids of the present invention.

### I. Probe Development Strategy

The first step taken in the development of the probes of the present invention involved identification of regions of the 16S rRNA which potentially could serve as target sites for specific nucleic acid probes with the desired sensitivity. This desired sensitivity included finding probe targets unique to Mycoplasma fermentans and thus by analogy the VLIA agents of Lo or Montagnier. For this analysis, precise alignments of mycoplasma 16S rRNA sequences were developed. The essentially complete 16S rRNA sequences of Mycoplasma fermentans as well as the 16S rRNA sequences of M*.* genitalium, M. hominis, M. agalactiae, M. arginini, M. arthritidis, M. bovigenitalium, M. californicum, M. capricolum, M. ellychniae, M. melaleucum, M. gallisepticum, M. hyopneumoniae, M. hyorhinis, M. iowae, M. lipophilum, M. mobile, M. muris, M. mycoides, M. neurolyticum, M. orale, M. pneumoniae, M. pulmonis, M. putrefaciens, M. salivarium, M. sualvi, ten Spiroplasma species' sequences, four Acholeplasma species' sequences, four Anaeroplasma species' sequences, Ureaplasma urealyticum, and one Asteroleplasma sequence were developed. For consideration of other microorganisms capable of deep tissue morbidity, rRNA sequences from several Candida yeasts, Neisseria meningitidis, Staphylococcus aureus, Escherichia coli, and numerous other species were utilized.

Four nucleic acid sequences defined by probes 2190, 2193, 2261 and 2273 exhibit exclusivity and inclusivity characteristics toward Mycoplasma fermentans. Probe 2190 is directed to a region of the 16S rRNA extending from nucleotide locations 50 to 100. Probe 2193 is directed to a region of the 16S rRNA extending from 130 to 165. Probe 2261 is directed to a region of the 16S rRNA extending from 160 to 200. Probe 2273 is directed to a region of the 16S rRNA extending from 420 to 470. The exclusivity and inclusivity characteristics of the nucleic acid defined by probes 2190, 2193, 2261 and 2273 would be maintained by nucleic acids sharing homology or complementarity to at least 90% of a ten nucleotide sequence within the probes.

The nucleic acids of the present invention are not to be construed as restricted to the precise specific nucleotides of the named probes. For example, the length of these particular oligonucleotides was optimized for use in the dot blot and sandwich assays. Optimal probe length will be a function of the hybridization conditions chosen and each of the conditions can be altered accordingly. In considering sets comprised of more than one nucleic acid, it is desirable that all probes behave in a compatible manner in any particular format in which they are employed. Thus, the exact length of a particular nucleic acid will to a certain extent reflect its specific intended use.

The nucleic acids of the present invention can be employed as oligonucleotide probes, or can be incorporated into larger polynucleotides of either ribonucleic acid or deoxyribonucleic acid. Sequences complementary to the probes described herein can be used as probes to rRNA genes. The preferred probes or their complements can be employed as chain elongation initiators for polymerase chain reaction, sequencing or other applications.

### Example 1. Dot-Blot Analysis of Probe Hybridization Behavior

Dot-blot analysis, in accordance with well known procedures, involves immobilizing a nucleic acid or a population of nucleic acids on a filter such as nitrocellulose, nylon, or other derivatized membranes which can readily be obtained commercially, specifically for this purpose. Either DNA or RNA can be easily immobilized on such a filter and subsequently probed or tested for hybridization under any of a variety of conditions (i.e., stringencies) with nucleotide sequences or probes of interest. Under stringent conditions, probes whose nucleotide sequences have greater complementarity to the target will exhibit a higher level of hybridization than probes containing less complementarity.

Probes of the present invention were tested in a dot-blot format. One hundred nanograms of RNA, purified by phenol extraction and ethanol precipitation (in some cases, also purified by cesium trifluoroacetate ultracentrifugation) was denatured and spotted on a nylon membrane. Probes were isotopically labelled with the addition of a ³²Phosphorous moiety to the 5' end of the oligonucleotide. Hybridization of probes occurred; at 60°C in the presence of 1.08 M sodium chloride, 60 mM sodium phosphate, and 6 mM ethylenediamine tetraacetic acid, pH 7.4. Unhybridized probe was removed by washing at a salt concentration one-third of the hybridization condition. The filters were exposed to X-ray film and the intensity of hybridization signals was evaluated after three hours of exposure.

Table 2 summarizes the behavior of the probes as employed in the present example, and documents the specificity of the probes of the present invention.

The representative strain of M. fermentans is the type strain, PG18 (American Type Culture Collection--ATCC 1989). Additional species of bacteria and a few fungi were added to the panel in order to represent the breadth of known bacterial taxa.

In Table 2, "++++" represents strongest hybridization signal after three hours exposure, with slightly lighter signal represented by "+++", diminishing to "++", then "+". "+-" means that the signal is virtually absent, and "-" is indicative of no hybridization of probe to target.

### Example 2. Dual Probe Hybridization

The nucleic acids of the present invention have utility in sandwich hybridization assays. Turning now to Figure 1, a sandwich assay is described which features a capture probe 12, a detector probe 13 and a solid support 10. The capture probe 12 ideally would be a bifunctional polynucleotide manufactured by adding a homopolymeric 3' tail to a probe with high target specificity. The tail would, in turn, hybridize to the complimentary homopolymer 11 on a solid surface 10, such as a glass bead or a filter disc. Hybridization of the capture probe 12 to its target 15, in this case Mycoplasma fermentans rRNA or rDNA, would complex the target 15 with the solid support 10. The detector probe 13, advantageously also with some degree of specificity, would be part of a detection scheme relying on radioactivity, fluorescence, chemiluminescence, color. As illustrated, detection probe 13 features a detection moiety 14 which would report the presence of the entire hybridization complex. The detector probe could potentially be incorporated as an RNA sequence into an amplifiable Q-beta midivariant as described by Kramer and Lizardi (Nature, vol. 339, 1989).

### Example 3. Clinical diagnosis of Mycoplasma fermentans or VLIA infection

A clinical sample, blood, a swab, urine, lavage, tissue, or cerebrospinal fluid is processed so as to liberate the total nucleic acid content. The sample, containing disrupted mycoplasmas is incubated in the presence of capture probe, detector probe, and magnetic particle beads which have been derivatized with oligo-Thymidine--as in Example 2--in a chaotropic buffer such as guanidinium isothiocyanate.

If target molecules, i.e., Mycoplasma fermentans or VLIA 16S rRNA or rDNA, are present in a given sample, a Bead + Capture Probe + Target + Detector Probe hybridization complex is formed, as in Figure 1. The presence of a magnet near the bottom of the reaction tube will cause the magnetic particle + hybridization complex to adhere to the side of the tube, enabling removal of the sample matrix, unbound probe, etc. Repeated rehydration and denaturation of the bead+probe+target complex would enable significant background reduction (as described in USSN 922,155, Collins, 1986). In this example, final detection could entail spotting the beads on membrane and assaying by autoradiography. Alternatively, the detector probe could be an amplifiable midivariant probe as described in Example 2.

For this particular assay, the following capture and detector probes are examples of preferred pairs:
Probes 2193 + 2261
Probes 2193 + 2190
Probes 2261 + 2190
Probes 2261 + 2273

### Example 4. Clinical diagnosis of Mycoplasma fermentans or VLIA infection from human sample employing polymerase chain reaction amplification of mycoplasma rDNA

Sample processing is designed so as to yield DNA. One of the probes described herein is used in conjunction with the antiparallel complement of one of the probes described herein to enzymatically amplify a segment of Mycoplasma fermentans or VLIA gene encoding mycoplasma rRNA in a polymerase chain reaction. Resultant material can then be assayed in a "sandwich" hybridization (Example 2) with any of the probes described herein. The polymerase chain reaction can, itself, be made either highly specific by employing probe/ primers described herein, or the reaction can be made more general using probes such as those described in copending USSN 359,158, and then identifying the amplification product as M. pneumoniae as described in Example 2. Probe 2261 ana the reverse complement of Probe 2190 could be used to make a highly specific test for the presence of M. fermentans/VLIA followed by a confirmatory hybridization employing Probe 2193. Resultant material can then be assayed in a "sandwich" assay.

### Example 5. In situ hybridization as a cytological stain

The probes of the present invention can ce employed as cytological staining reagents specific for Mycoplasma fermentans. For example, a blood or tissue specimen is applied to a microscope slide. After appropriate fixation and lysis, hybridization of probes is carried out in situ. In this example, mycoplasmas could be visualized in a specimen by fluorescently labelling Probe 2193 or 2261 and examining the slide using a fluorescent microscope, looking for small fluorescent bodies.

### Example 6. Confirmation of presumptive mycoplasma species identification following culture

Following a standard cultivation step for Mycoplasma fermentans, or VLIA, for example on H-agar plates. SP-6 broth. 10B broth, or A8 agar, a colony or liquid culture is tested for the presence of Mycoplasma fermentans or VLIA by employing sandwich hybridization assays as described in Examples 2 and 3.

## Claims

1. A nucleic acid consisting of a sequence of nucleotides that is complementary or homologous to all or at least ten consecutive nucleotides in a region of the 16S rRNA of *Mycoplasma fermentans,* which region is selected from the group of regions consisting of 50 to 100, 130 to 165, 160 to 200, and 420 to 470.

2. A nucleic acid of claim 1, wherein said sequence of nucleotides is complementary or homologous to a sequence comprising any ten consecutive nucleotides within a sequence selected from the group of sequences defined by probes 2190, 2193, 2261, and 2273.

3. A nucleic acid of claim 1, wherein said sequence of nucleotides is complementary or homologous to a sequence selected from the group of sequences defined by probes 2190, 2193, 2261, and 2273.

4. A set of nucleic acids comprising at least two different nucleic acids of claim 1.

5. A set of nucleic acids comprising at least two different nucleic acids of claim 2.

6. A set of nucleic acids of claim 5, wherein said set comprises any one of probe pairs:
2193 and 2261:
2193 and 2190:
2261 and 2190; and
2261 and 2273.

7. A method for detecting the presence of *Mycoplasma fermentans* in a sample comprising:
a) contacting the sample with at least one nucleic acid of claim 1;
b) imposing hybridization conditions on the sample and said nucleic acid to allow said nucleic acid to hybridize to the rRNA or rDNA of *Mycoplasma fermentans,* if present, in the sample, to form a stable nucleic acid complex under conditions which do not allow said nucleic acid to form a stable nucleic acid complex with rRNA or rDNA of non-*Mycoplasma fermentans* organisms; and
c) detecting said nucleic acid complex as an indication of the presence of *Mycoplasma fermentans* in the sample.

8. A method of claim 7, wherein said nucleic acid of said contacting step is complementary or homologous to a sequence comprising any ten consecutive nucleotides within a sequence selected from the group of sequences defined by probes 2190, 2193, 2261, and 2273.

9. A method of claim 7, wherein a set of at least two nucleic acids is used in said contacting step, each nucleic acid in said set consisting of a sequence of nucleotides that is complementary or homologous to a sequence comprising any ten consecutive nucleotides within a sequence selected from the group of sequences defined by probes 2190, 2193, 2261, and 2273.

10. A method of claim 9, wherein said set of nucleic acids comprises any one of probe pairs:
2193 and 2261:
2193 and 2190;
2261 and 2190; and
2261 and 2273.

11. A kit for detecting pathogenic *Mycoplasma fermentans,* said kit comprising a nucleic acid of claim 1.

12. A nucleic acid probe for detecting *Mycoplasma fermentans,* said probe comprising a nucleic acid of claim 1, and one or more of an end cap, a detection moiety, a homopolymer nucleic acid, and an amplifiable Q-beta midivariant.

## Patentansprüche

1. Nucleinsäure, bestehend aus einer Nucleotidsequenz, die komplementär oder homolog zu sämtlichen oder mindestens zehn aufeinanderfolgenden Nucleotiden in einem Bereich der 16S rRNA von *Mycoplasma fermentans* ist, der aus der Gruppe von Bereichen, bestehend aus 50 bis 100, 130 bis 165, 160 bis 200 und 420 bis 470, ausgewählt ist.

2. Nucleinsäure nach Anspruch 1, dadurch **gekennzeichnet**, daß die Nucleotidsequenz komplementär oder homolog zu einer Sequenz ist, die zehn aufeinanderfolgende Nucleotide innerhalb von Sequenzen, ausgewählt aus der Gruppe von Sequenzen, definiert durch Sonden, bestehend aus 2190, 2193, 2261 und 2273, umfaßt.

3. Nucleinsäure nach Anspruch 1, dadurch **gekennzeichnet**, daß die Nucleotidsequenz komplementär oder homolog zu einer Sequenz, ausgewählt aus der Gruppe von Sequenzen, definiert durch Sonden, bestehend aus 2190, 2193, 2261 und 2273, ist.

4. Satz von Nucleinsäuren, umfassend mindestens zwei verschiedene Nucleinsäuren nach Anspruch 1.

5. Satz von Nucleinsäuren, umfassend mindestens zwei verschiedene Nucleinsäuren nach Anspruch 2.

6. Satz von Nucleinsäuren nach Anspruch 2, dadurch **gekennzeichnet**, daß der Satz eines der Sondenpaare:
2193 und 2261;
2193 und 2190;
2261 und 2190; und
2261 und 2273
umfaßt.

7. Verfahren zum Nachweis von *Mycoplasma fermentans* in einer Probe, umfassend:
a) Kontaktieren der Probe mit mindestens einer Nucleinsäure nach Anspruch 1;
b) Belegen der Probe und der Nucleinsäure mit Hybridisierungsbedingungen, um die Nucleinsäure mit der gegebenenfalls in der Probe vorhandenen rRNA oder rDNA von *Mycoplasma fermentans* zu hybridisieren, um einen stabilen Nucleinsäurekomplex unter Bedingungen zu bilden, unter denen die Nucleinsäure keinen stabilen Nucleinsäurekomplex mit rRNA oder rDNA von anderen Organismen als *Mycoplasma fermentans* bildet; und
c) Nachweis des Nucleinsäurekomplexes als Hinweis auf das Vorliegen von *Mycoplasma fermentans* in der Probe.

8. Verfahren nach Anspruch 7, dadurch **gekennzeichnet**, daß die Nucleinsäure der Kontaktierungsstufe komplementär oder homolog zu einer Sequenz, umfassend zehn aufeinanderfolgende Nucleotide innerhalb von Sequenzen, ausgewählt aus der Gruppe von Sequenzen, definiert durch Sonden, bestehend aus 2190, 2193, 2261 und 2273, ist.

9. Verfahren nach Anspruch 7, dadurch **gekennzeichnet**, daß ein Satz von mindestens zwei Nucleinsäuren in der Kontaktierungsstufe verwendet wird, wobei jede Nucleinsäure in dem Satz aus einer Nucleotidsequenz besteht, die komplementär oder homolog zu einer Sequenz ist, welche zehn aufeinanderfolgende Nucleotide innerhalb von Sequenzen, ausgewählt aus der Gruppe von Sequenzen, definiert durch Sonden, bestehend aus 2190, 2193, 2261 und 2273, umfaßt.

10. Verfahren nach Anspruch 9, dadurch **gekennzeichnet**, daß der Satz von Nucleinsäuren eines der Sondenpaare
2193 und 2261;
2193 und 2190;
2261 und 2190; und
2261 und 2273
umfaßt.

11. Kit zum Nachweis von pathogenem *Mycoplasma fermentans,* dadurch **gekennzeichnet**, daß der Kit eine Nucleinsäure nach Anspruch 1 umfaßt.

12. Nucleinsäuresonde zum Nachweis von *Mycoplasma fermentans,* dadurch **gekennzeichnet**, daß die Sonde eine Nucleinsäure nach Anspruch 1 und eine End-Cap-Struktur, eine Detektionsgruppierung, eine homopolymere Nucleinsäure und/oder eine amplifizierbare Q-beta Midivariante umfaßt.

## Revendications

1. Acide nucléique constitué d'une séquence de nucléotides qui est complémentaire ou homologue à tous les nucléotides ou au moins à dix nucléotides successifs dans une région de l'ARNr 16S de *Mycoplasma fermentans,* ladite région étant sélectionnée parmi le groupe de régions constitué par les régions 50 à 100, 130 à 165, 160 à 200 et 420 à 470.

2. Acide nucléique selon la revendication 1, dans lequel ladite séquence de nucléotides est complémentaire ou homologue à une séquence comprenant dix nucléotides successifs quels qu'ils soient dans des séquences sélectionnées parmi le groupe de séquences définies par des sondes constituées par les sondes 2190, 2193, 2261 et 2273.

3. Acide nucléique selon la revendication 1, dans lequel ladite séquence de nucléotides est complémentaire ou homologue à une séquence choisie parmi le groupe de séquences définies par des sondes constituées par les sondes 2190, 2193, 2261 et 2273.

4. Groupe d'acides nucléiques comprenant au moins deux acides nucléiques différents selon la revendication 1.

5. Groupe d'acides nucléiques comprenant au moins deux acides nucléiques différents selon la revendication 2.

6. Groupe d'acides nucléiques selon la revendication 2, dans lequel ledit groupe comprend n'importe quelles paires de sondes:
2193 et 2261;
2193 et 2190;
2261 et 2190; et
2261 et 2273.

7. Procédé pour détecter la présence de *Mycoplasma fermentans* dans un échantillon, comprenant le fait de:
a) mettre l'échantillon en contact avec au moins un acide nucléique selon la revendication 1,
b) imposer des conditions d'hybridation à l'échantillon et audit acide nucléique pour permettre audit acide nucléique de s'hybrider à l'ARNr ou à l'ADNr de *Mycoplasma fermentans* s'il est présent dans l'échantillon pour former un complexe stable d'acide nucléique dans des conditions qui ne permettent pas audit acide nucléique de former un complexe stable d'acide nucléique avec l'ARNr ou l'ADNr d'organismes différents de *Mycoplasma fermentans,* et
c) détecter ledit complexe d'acide nucléique comme étant une indication de la présence de *Mycoplasma fermentans* dans l'échantillon.

8. Procédé selon la revendication 7, dans lequel ledit acide nucléique de ladite étape de mise en contact est complémentaire ou homologue à une séquence comprenant dix nucléotides successifs quels qu'ils soient dans des séquences sélectionnées parmi le groupe de séquences définies par des sondes constituées par les sondes 2190, 2193, 2261 et 2273.

9. Procédé selon la revendication 7, dans lequel on utilise un groupe d'au moins deux acides nucléiques dans ladite étape de mise en contact, chaque acide nucléique dans ledit groupe étant constitué d'une séquence de nucléotides qui est complémentaire ou homologue à une séquence comprenant dix nucléotides successifs quels qu'ils soient dans des séquences sélectionnées parmi le groupe de séquences définies par des sondes constituées par les sondes 2190, 2193, 2261 et 2273.

10. Procédé selon la revendication 9, dans lequel ledit groupe d'acides nucléiques comprend n'importe quelles paires de sondes:
2193 et 2261;
2193 et 2190;
2261 et 2190; et
2261 et 2273.

11. Nécessaire pour détecter le microbe pathogène *Mycoplasma fermentans,* ledit nécessaire comprenant un acide nucléique selon la revendication 1.

12. Sonde d'acide nucléique pour détecter *Mycoplasma fermentans,* ladite sonde comprenant un acide nucléique selon la revendication 1 et un ou plusieurs des éléments ci-après: une coiffe terminale, une fraction de détection, un acide nucléique homopolymère et un midivariant Q-bêta amplifiable.
